# EUROPEAN PATENT SPECIFICATION

(11) **EP 3 978 050 B1**
(45) Date of publication and mention of the grant of the patent: **02.07.2025**
(21) Application number: 20818464.8
(22) Date of filing: 27.05.2020
(51) Int. Cl.: A61M 5/315, A61M 5/31

(54) **GASKET, SYRINGE PROVIDED WITH SAME, AND METHOD FOR PRODUCING GASKET**
DICHTUNG, SPRITZE DAMIT UND VERFAHREN ZUR HERSTELLUNG EINER DICHTUNG
JOINT, SERINGUE POURVUE DE CELUI-CI, ET MÉTHODE DE PRODUCTION D'UN JOINT

(30) Priority: 03.06.2019 JP 2019104030
(43) Date of publication of application: 06.04.2022
(73) Proprietor: Coki Engineering Inc., Osaka-shi, Osaka 540-0037 (JP)
(72) Inventor: YOTSUTSUJI Akira, Osaka-shi, Osaka 540-0037 (JP)
(74) Representative: HKW Intellectual Property PartG mbB
(86) International application number: PCT/JP2020/020974
(87) International publication number: WO 2020/246343

(56) References cited:
- EP-A1- 2 703 025
- EP-A1- 2 926 851
- WO-A1-2012/101669
- JP-A- 2001 190 667
- JP-A- 2001 190 667
- JP-A- 2014 047 828
- JP-A- 2014 213 092
- JP-A- 2016 022 269
- JP-A- 2019 013 537
- JP-A- S5 452 889
- JP-U- S 488 990
- US-A1- 2014 319 778
- US-A1- 2018 344 940
- US-A1- 2018 344 940
- US-A1- 2019 125 976

## Description

### BACKGROUND OF THE INVENTION

### Field of the Invention

The present invention relates to a gasket for a syringe, used in administering a medical solution to a human body or an animal in pharmaceutical and medical fields, a syringe including the gasket, and a method of manufacturing the gasket.

### Background Art

There has been conventionally proposed a gasket exerting high safety and high sealing performance over a long period of time, with a medical solution being injected therein (see e.g., Publication of Japanese Translation of PCT International Application No. JP 2004-525011 A).

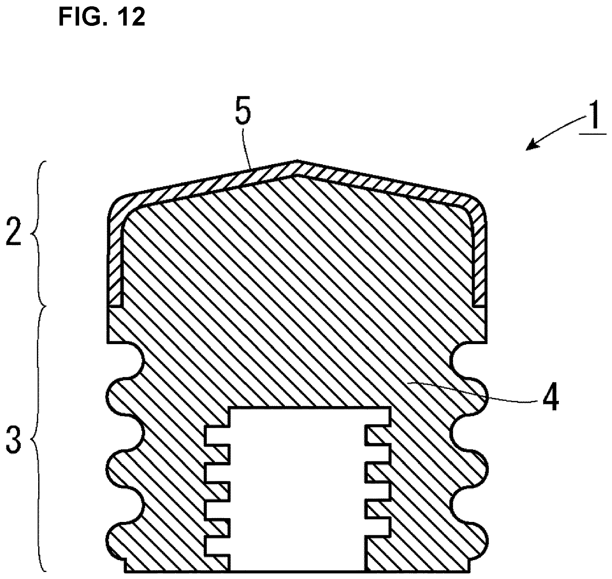

As shown in FIG. 12, a gasket 1 disclosed in Publication of Japanese Translation of PCT International Application No. JP 2004-525011 A mainly includes a gasket body 4, composed of a plunger section 2 and a seal section 3, and an inert film 5 laminated on the surface of the plunger section 2 of the gasket body 4.

The inert film 5 exerts high resistance against medical solution and is thus laminated on the plunger section 2 of the gasket body 4, whereby the gasket 1 per se can be enhanced in resistance against medical solution.

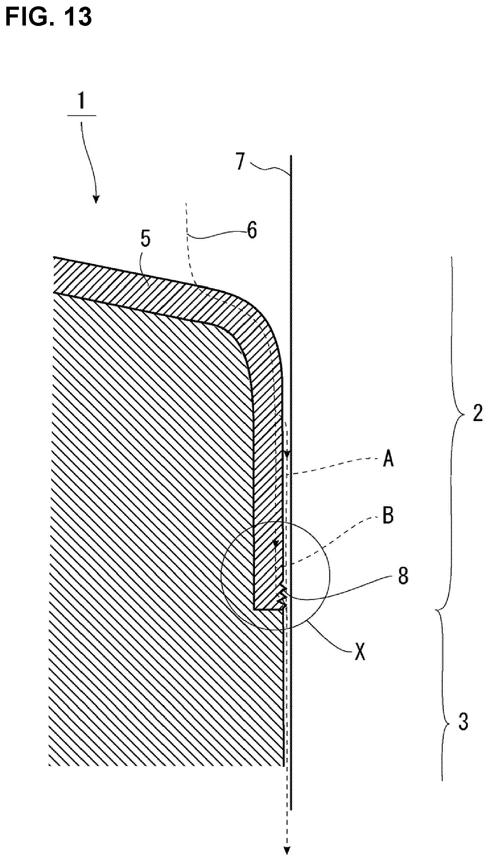

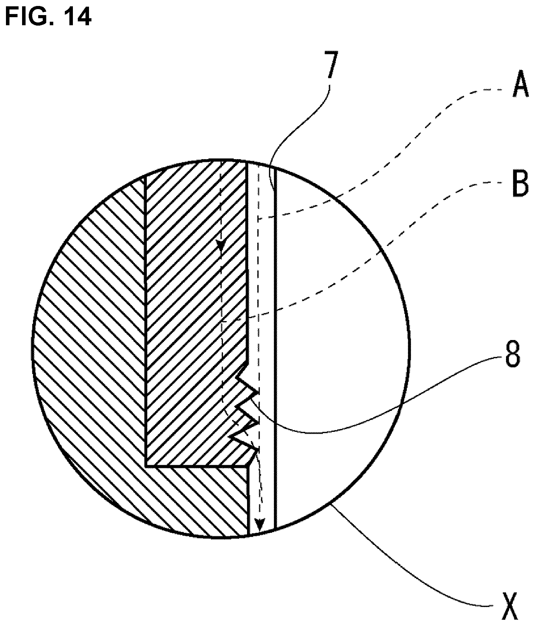

In spite of the advantage described above, the well-known gasket 1 has had a drawback as follows. When a certain time elapses after setting the gasket 1 in a syringe 7 in which a medical solution 6 is encapsulated, the medical solution 6 seeps through the inert film 5 and leaks out as shown in FIGS. 13 and 14. This occurs due to a structure that a cut surface 8 of the inert film 5 is located on the lateral surface of the gasket 1.

As a reason for the drawback, it has been found that a large number of fine closed cells are formed inside and on the surface of a polytetrafluoroethylene (PTFE) film used as the inert film 5 (e.g., Japan Laid-open Patent Application Publication No. 2013-154264). The PTFE film is stretched to a great extent, when adhered to the surface of the plunger section 2 of the gasket body 4. Undesirably, the closed cells are connected to each other in alignment, whereby fine "pathways" (communicating holes) are formed. The medical solution 6 (especially, a surfactant-containing medical solution having become popular in recent years) gradually penetrates the pathways, and as a result, seeps therethrough and leaks out (see a leaking-out route "A" in FIGS. 13 and 14).

Furthermore, according to a manufacturing procedure of the gasket 1 disclosed in Publication of Japanese Translation of PCT International Application No. JP 2004-525011 A, the inert film 5 is configured to be cut after molding the gasket body 4, whereby the cut surface 8 of the inert film 5 is exposed to the lateral surface of the gasket 1. Because of this, when permeating into the inert film 5 due to the reason described above, the medical solution 6 could leak out in large amount from the cut surface 8 through the communicating holes described above (see a leaking-out route "B" in FIGS. 13 and 14).

The present invention has been developed in view of the drawback of the prior arts described above. Hence, it is a main object of the present invention to provide a gasket, by which undesirable leakage of a medical solution is unlikely to occur, for instance, when the gasket is set in a syringe pre-filled with the medical solution and contacts the medical solution over a long period of time, a syringe including the gasket, and a method of manufacturing the gasket.

EP 2 703 025 A1 discloses a gasket for glass or resin syringes which is laminated with an inert resin film and is excellent in plugging properties, liquid-tightness, and slidability. The gasket laminated with an inert resin film has multiple circular ribs that are to be in sliding contact with an inner wall of a syringe barrel, wherein the circular ribs include a front circular rib having a sliding contact portion whose cross section has a diameter that expands substantially linearly toward a back end of the syringe barrel, the linear portion has a length of 10 to 55% of the length of a sliding side face of the gasket, and the diameter of the linear portion which expands substantially linearly toward a back end of the syringe barrel has a diameter expansion ratio X between a minimum diameter r (mm) and a maximum diameter R (mm) of 0.1 to 8.0%.

JP 2001 190667 A discloses a gasket comprising a distal, first molded part 1 containing a drug-contact surface and a first cyclic sliding surface which is adjacent to this, and a proximal, second molded parts 2 containing a plurality of cyclic sliding surfaces and formed by insert molding of the first molded part 1. The lateral surface containing the drug-contact surface and the first cyclic sliding surface of the first molded part 1 is laminated with a synthetic resin film, and the end surface of the film laminated on the lateral surface of the first molded part 1 is implanted in the second molded part 2.

EP 2 926 851 A1 discloses a method for producing a prefilled syringe gasket, which ensures that a sliding portion of the gasket (a portion of the gasket to be kept in contact with a syringe barrel) is substantially free from a scratch or an existing scratch of the gasket is substantially eliminated. The prefilled syringe gasket includes a liquid contact portion, and a sliding portion to be kept in contact with an inner surface of a syringe barrel. The method includes the steps of: forming the gasket; and rubbing a surface of the sliding portion of the formed gasket against a predetermined base, wherein a rubbing direction in which the surface of the sliding portion is rubbed in the rubbing step extends circumferentially of the sliding portion.

US 2014 / 319778 A1 discloses a gasket device for use in a pre-filled syringe having a cylinder for containing liquid drug in sealing tightness. The liquid drug is pressurized by moving a plunger in the cylinder. The gasket device includes a gasket body, having resiliency, and movable toward the liquid drug with a front end of the plunger. A laminate layer is formed from PTFE (polytetrafluoroethylene), and disposed to cover a surface of the gasket body. The laminate layer includes a sealing portion, having a thickness Da, for sliding on an inner surface of the cylinder in sealing tightness. A liquid receiving portion has a thickness Db, for contacting the liquid drug in the cylinder. The thickness Da is from 10 microns to 40 microns. The thickness Db is from 20 microns to 50 microns. A ratio R1 of the thickness Db to the thickness Da is from 1.25 to 2.00.

US 2018 / 344940 A1 discloses a method of manufacturing a sealing element for use in a fluid conveyance device comprising the steps of: forming an elastomeric sealing body including a first body portion defining at least one annular sealing rib, and a second body portion arranged on an end of the first body portion, the second body portion comprising an annular protrusion extending radially beyond the first body portion; and forming a layer of barrier material on a free end of the second body portion, wherein the forming steps are carried out in a single molding operation.

WO 2012 / 101669 A1 discloses a gasket optimized for a pre-filled syringe, which has high sliding performance on a cylinder by using a sliding film, deals with a problem of an existing gasket main body formed of isobutylene-isoprene rubber by using a gasket main body formed of silicon rubber, and minimizes a concern that a liquid medicine in the cylinder (or moisture in the liquid medicine) may be dissipated to outside air and deals with a problem caused by permeability of the silicon rubber by covering a rear side of the gasket main body (an inner side surface of a concavity, and a rear-side circumferential edge portion near the concavity) with a non-permeable plastic base body and a flange portion.

### SUMMARY OF THE INVENTION

The invention is defined by the subject-matter of independent claims 1, 6 and 7. Further embodiments are defined in the dependent claims.

According to an aspect of the present invention, a gasket is provided that includes the features of claim 1

Preferably, the circumferential end surface of the PTFE film is made sealed by the gasket body.

Preferably, the circumferential end portion of the PTFE film attached to the solution contact surface of the gasket body has an extension ratio of less than or equal to 10%.

Preferably, the gasket body is made of silicone rubber provided with slidability.

According to yet another aspect of the present invention, it is intended to provide a syringe that includes the gasket configured as any of the above, a medical solution, a syringe barrel, and a plunger rod.

According to further yet another aspect of the present invention, a method of manufacturing a gasket is provided that includes the features of claim 6Accordingly, a circumferential end portion of the PTFE film is curved to cover a circumferential edge of the solution contact surface, while a circumferential end surface of the PTFE film is buried in the slide contact portion of the gasket body so as not to be exposed to outside and is made sealed by the gasket body.

According to still further yet another aspect of the present invention, a method of manufacturing a gasket is provided that includes the features of claim 7 Accordingly, a circumferential end portion of the PTFE film is curved to cover a circumferential edge of the slide contact portion, while a circumferential end surface of the PTFE film faces in surface contact with a lateral surface of the gasket body and is made sealed by the gasket body.

### Advantageous Effects of Invention

According to the gasket of the present invention, the circumferential end portion of the PTFE film is curved toward the slide contact portion of the gasket body, and the circumferential end surface of the PTFE film is buried in the slide contact portion of the gasket so as not to be exposed to the outside. Thus, the circumferential end surface of the PTFE film is not exposed to the outside, whereby it is possible to prevent occurrence of an undesirable situation that a medical solution penetrates the PTFE film and leaks out from the circumferential end surface thereof through communicating holes and this situation brings about leakage of the medical solution from the gasket. Furthermore, when the circumferential end surface is made sealed by the gasket body, it is possible to increase as much as possible the possibilities of preventing leakage of the medical solution from the gasket.

Accordingly, it is possible to provide a gasket, by which undesirable leakage of a medical solution is unlikely to occur, for instance, when the gasket is set in a syringe pre-filled with the medical solution and contacts the medical solution over a long period of time, a syringe including the gasket, and a method of manufacturing the gasket.

### BRIEF DESCRIPTION OF THE DRAWINGS

Referring now to the attached drawings which form a part of this original disclosure:
FIG. 1 is a cross-sectional view of a syringe (100) according to a practical example to which the present invention is applied;
FIG. 2 is a cross-sectional view of a gasket (10) according to the practical example to which the present invention is applied;
FIG. 3 is a cross-sectional view of a gasket body (12) according to the practical example to which the present invention is applied;
FIG. 4 is an enlarged cross-sectional view of a part A shown in FIG. 2 and shows a state of a PTFE film (4) attached to the gasket body (12);
FIG. 5 is a diagram showing a method of manufacturing the gasket (10);
FIG. 6 is a diagram showing the method of manufacturing the gasket (10);
FIG. 7 is a diagram showing the method of manufacturing the gasket (10);
FIG. 8 is an enlarged diagram showing the method of manufacturing the gasket (10);
FIG. 9 is a cross-sectional view of a gasket (10) according to a practical example to which a modification 1 is applied;
FIG. 10 is a diagram showing a method of manufacturing the gasket (10) according to the modification 1;
FIG. 11 is an enlarged diagram showing the method of manufacturing the gasket (10) according to the modification 1;
FIG. 12 is a cross-sectional view of a gasket (1) according to a prior art;
FIG. 13 is an enlarged cross-sectional diagram showing a state of the gasket (1) according to the prior art set in a syringe (7); and
FIG. 14 is an enlarged cross-sectional view of a part X shown in FIG. 13.

### DETAILED DESCRIPTION OF EMBODIMENTS

### (Configurations of Gasket 10 and Syringe 100)

A gasket 10, to which the present invention is applied, and a syringe 100 including the gasket 10 will be explained along with a practical example shown in drawings.

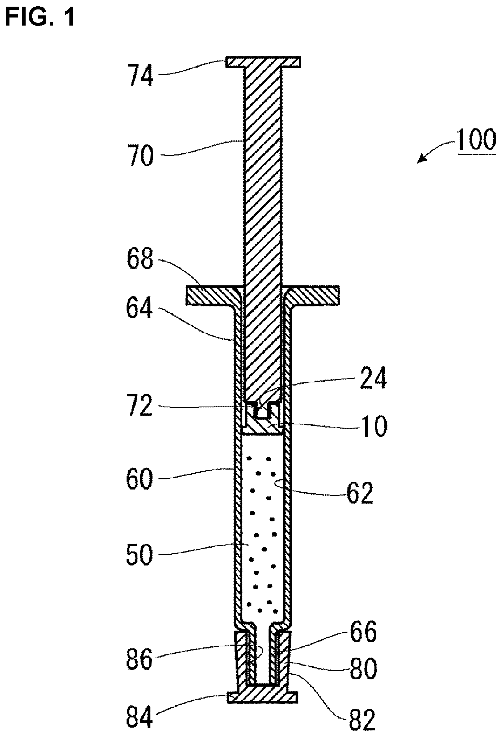

As shown in FIG. 1, the syringe 100 mainly includes the gasket 10, a medical solution 50, a syringe barrel 60, a plunger rod 70, and a top cap 80.

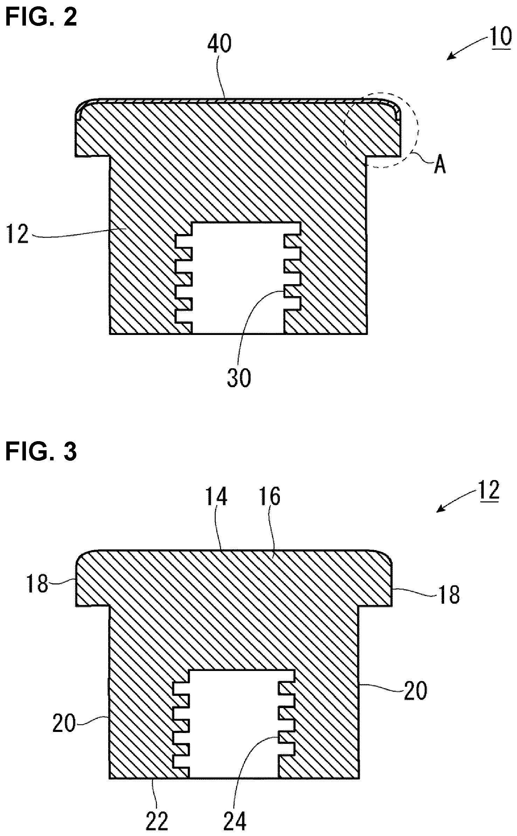

As shown in FIG. 2, the gasket 10 mainly includes a gasket body 12 and a polytetrafluoroethylene (PTFE) film 40.

The gasket body 12 has an approximately columnar shape. As shown in FIG. 3, the gasket body 12 includes a solution contact portion 16, a slide contact portion 18, and a small diameter portion 20. The solution contact portion 16 has a tip surface (referred to as "a solution contact surface 14" in the specification of the present application) that contacts the medical solution 50 when the gasket body 12 is fitted into the syringe barrel 60. The slide contact portion 18 is shaped in continuation to the solution contact portion 16 and has a diameter slightly greater than a diameter (inner diameter) of an inner surface 62 of the syringe barrel 60 into which the gasket 10 is fitted. The small diameter portion 20 is shaped in continuation to the slide contact portion 18 and is reduced in diameter to much extent than the slide contact portion 18.

Besides, the gasket body 12 is provided with a female threaded hole 24 in a rear end surface 22 thereof. The plunger rod 70 is attached to the female threaded hole 24.

A variety of elastomers (e.g., butyl rubber or silicone rubber) is usable as a material of which the gasket body 12 is made.

It should be noted that in use of a vulcanized molded rubber such as a butyl rubber, a silicone oil is required to be applied to the inner surface 62 of the syringe barrel 60 because of a slidability-related problem. Hence, it is preferred to use "silicone rubber" provided with slidability as the material of which the gasket body 12 is made.

"Silicone rubber" is a thermosetting elastomer, and for instance, uses "organopolysiloxane" in a liquid, grease, or clay state as a raw material thereof. "Organopolysiloxane" is a material that a methyl, vinyl, phenyl, or trifluoropropyl group is incorporated in a molecule, and which group should be incorporated in a molecule depends on requirements for special properties.

There exists plural types of "silicone rubber". Although any type of "silicone rubber" is usable in the present practical example, a peroxide crosslinked silicone rubber is herein used as an example. The peroxide crosslinked silicone rubber is formed by preparing liquid or grease "organopolysiloxane" that a vinyl group is incorporated in a molecule, adding thereto a necessary filling and a peroxide curing agent and then kneading the whole. Alternatively, an addition reaction silicone rubber can be exemplified. The addition reaction silicone rubber is formed by heating and curing two types of clay polysiloxane through chemical reactions using an organometallic compound of platinum, rhodium, tin, or so forth as a catalyst. One type of clay polysiloxane contains a vinyl group incorporated in a molecule, whereas the other type contains reactive hydrogen incorporated in the terminal of a molecule.

The silicone rubber provided with slidability is formed by, for instance, adding a peroxide functioning as a crosslinking agent to the liquid, grease, or clay organosiloxane prepared as a material (alternatively, adding a curing catalyst to the two types of clay polysiloxane described above), then adding thereto a predetermined amount of silicone oil, and kneading the whole with a kneader. Furthermore, a silica fine powder is added to the kneaded material by an appropriate amount (e.g., 25%) to adjust the hardness of the kneaded material on an as-needed basis. If still necessary, for example, an ultrahigh molecular weight polyethylene (PE) fine powder is added thereto by a predetermined amount.

A PE resin, forming fine particles of the fine powder, is an ultrahigh molecular weight resin (the average molecular weight thereof is, for instance, 1 to 3 million or greater and may reach 7 million). Such ultrahigh molecular weight particles are not permeable to water, and besides, do not adhere to almost anything. Moreover, the molecular weight of the ultrahigh molecular weight PE is too high; hence, the ultrahigh molecular weight PE does not melt even at a high temperature and is kept in a spherical form even when molded at a high pressure. The surface of the spherical ultrahigh molecular weight PE is relatively smooth but is in part observed as uneven. The particle diameter of the spherical ultrahigh molecular weight fine particles contained in the fine powder falls in a range of 10 to 300µm. More preferably, the particle diameter falls in a range of 20 to 50µm. The ultrahigh molecular weight fine particles herein used have an average particle diameter of 25pm, 30µm, or so forth, albeit depending on the grade thereof. When the particle size distribution of the ultrahigh molecular weight fine particles is wide, small diameter particles enter between large diameter particles and fill the gaps between the large diameter particles, whereby a close-packed state is realized. Now that the close-packed state is realized, the fine particles become impermeable to water. Hence, even if a water permeable silicone rubber base material or silicone oil is used, a medical use slidable silicone rubber of the present invention as a whole is supposed to exert quite low water permeability.

The silica fine powder is a type of powder using silica sand as a raw material and is mostly made of silica (SiO₂). The silica fine powder is added to an elastic material to adjust the hardness thereof.

A method of molding the gasket body 12 will be exemplified. A compression mold, enabling molding of the gasket body 12, is heated to an appropriate temperature. The PTFE film 40 is put between blocks of the mold, and the molding material described above (the silicone rubber formed by adding the silica powder, the silicone oil, and on an as-needed basis, the ultrahigh molecular weight PE powder to the raw material and then kneading the whole) is filled in the mold. In this condition, the mold is tightened and then heated and pressurized along with a predetermined procedure. Accordingly, thermal crosslinking advances in 1 to 10 minutes, whereby the gasket body 12 is obtained as an intended object. It should be noted that the gasket body 12 herein obtained is preferably subjected to secondary thermal treatment (annealing).

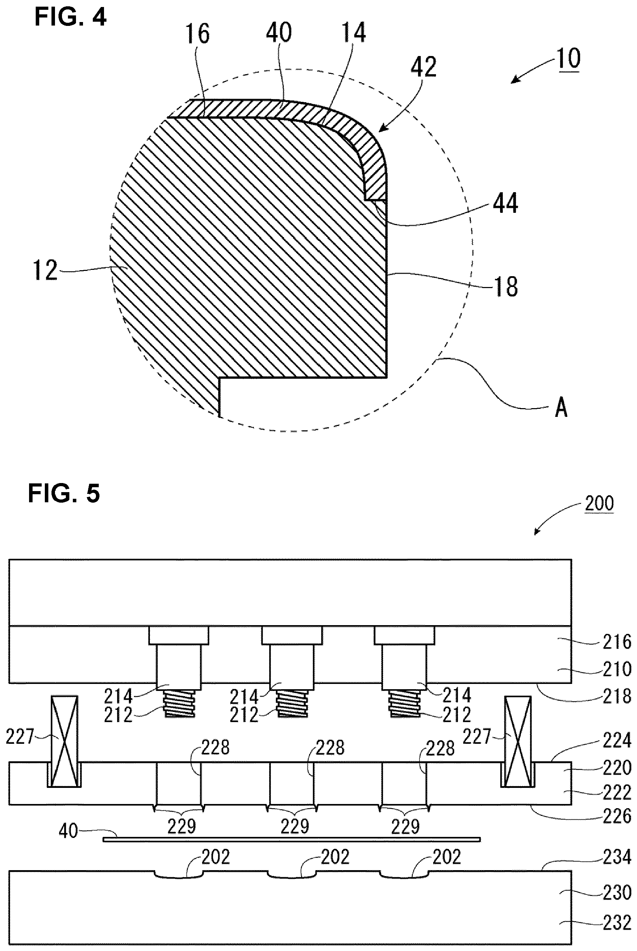

Next, the PTFE film 40 will be explained. As shown in FIG. 4, the PTFE film 40 is arranged and set to cover the solution contact surface 14 of the solution contact portion 16 of the gasket body 12. A circumferential end portion 42 of the PTFE film 40 is curved toward the slide contact portion 18 of the gasket body 12, whereby a circumferential end surface 44 of the PTFE film 40 is buried in the slide contact portion 18 of the gasket body 12 so as not to be exposed to the outside.

Pure PTFE may be used as the material of the PTFE film 40 in the present practical example. However, it is more preferred to use, for instance, modified PTFE mixed with 1 to 15% by mass of a fluorine resin (polytetrafluoroethylene-perfluoroalkyl vinyl ether copolymer (abbreviated as PFA), tetrafluoroethylene-hexafluoropropylene copolymer, etc.) functioning as a crystallization inhibitor for PTFE. This is because the PTFE film 40 is provided with elasticity when made of the modified PTFE.

Besides, not only the pure or modified PTFE described above but also a type of PTFE manufactured with "skived method" may be used as the PTFE film 40 in the present practical example. In the skived method, a sheet of PTFE is obtained by cutting a PTFE block (round bar) in which closed cells are formed by hot isostatic pressing called HIP treatment.

A primary sintered block of PTFE is obtained by compression-molding a powder of the pure or modified PTFE and then sintering the compression-molded powder. In this sintering, the particles of the powder are in close contact with each other at contact portions thereof; however, when the sintered block of PTFE is viewed as a whole, ultrafine gaps are produced between non-contact portions of the powder particles and continue to each other, whereby a minute fluid is enabled to pass therethrough.

Alternatively, "cast method" may be employed as another method of manufacturing the PTFE film 40. In the cast method, a sheet of PTFE is obtained by applying an emulsion of PTFE to the surface of a flat board so as to form a film thereon and then heating the film.

Referring back to FIG. 1, the syringe barrel 60 is a cylindrical container and is provided with an attached portion 66 and a flange portion 68. The attached portion 66 protrudes from the front end (tip) of a barrel body 64 and enables a syringe needle (not shown in the drawing) to be attached thereto. The flange portion 68 is a finger-hooked portion provided on the rear end of the barrel body 64. Not only glass but also a hard resin (e.g., cyclo olefin polymer (COP), polypropylene (PP), ethylene norbornene copolymer (COC), etc.) is used as the material of the syringe barrel 60. As described below, the gasket 10 according to the present practical example can keep high the watertightness of the syringe barrel 60 due to the structural feature thereof. Hence, a glass syringe barrel is also usable as the syringe barrel 60, albeit inferior to a resin syringe barrel in dimensional accuracy of the inner diameter.

The plunger rod 70 is a rod-shaped member. The plunger rod 70 is provided with a male threaded portion 72 on the front end (tip) thereof, while being provided with a finger pushing portion 74 on the rear end thereof. The male threaded portion 72 of the plunger rod 70 is shaped as a male threaded screw enabled to be screwed and fitted into the female threaded hole 24 provided in the gasket body 12 of the gasket 10. It should be noted that a resin such as cyclic polyolefin, polycarbonate, or polypropylene can be used as the material of the plunger rod 70.

The top cap 80 includes a cap body 82 made in shape of a conical frustum and a cap flange portion 84 laterally extending in a disc shape from the edge of the top surface of the cap body 82. The cap body 82 is provided with a recess 86 into which the attached portion 66 of the syringe barrel 60 is fitted. Besides, the top cap 80 may be made of an elastomer and a medical solution resistance film (of PTFE or PFA) may be laminated on the inner peripheral surface thereof. Here, the elastomer refers to a vulcanized rubber, a thermosetting elastomer, or a thermoplastic elastomer.

### (Manufacturing Procedure of Gasket 10)

Next, a manufacturing procedure of the gasket 10 according to the present preferred embodiment will be explained. First, a mold 200 to be used for manufacturing the gasket 10 will be explained.

For example, as shown in FIG. 5, the mold 200 is roughly divided into three blocks (a first block 210, a second block 220, and a third block 230). The first block 210 is provided with plural pairs of a piston 214 and a male threaded screw 212 corresponding to the female threaded hole 24 formed in the gasket body 12. Each pair of the piston 214 and the male threaded screw 212 protrudes downward from a first parting surface 218 located on the lower surface of a first block body 216 of the first block 210. First, the piston 214 is formed to protrude from the first parting surface 218, and then, the male threaded screw 212 is formed to protrude from the lower end of the piston 214.

The second block 220 is provided with plural molding elastomer block fitting holes 228 in a second block body 222 thereof. The molding elastomer block fitting holes 228 penetrate the second block body 222 from a second upper parting surface 224 thereof located on the upper side in FIG. 5 to a second lower parting surface 226 thereof located on the lower side in FIG. 5. The molding elastomer block fitting holes 228 are holes into which plural molding elastomer blocks 204 to be molded into plural gasket bodies 12 are fitted. It should be noted that each molding elastomer block 204 is fitted into each molding elastomer block fitting hole 228 such that the solution contact portion 16 of the gasket body 12 obtained by molding each molding elastomer block 24 faces downward in FIG. 5.

Besides, each molding elastomer block fitting hole 228 is provided with a cutting portion 229 on a second lower parting surface 226-side end thereof. The cutting portion 229 faces the third block 230. As described below, an uncut PTFE film 40 is configured to be cut between the cutting portion 229 and the third block 230.

Moreover, the second upper parting surface 224 is provided with plural springs 227 protruding toward the first block 210.

The third block 230 is provided with plural molding recesses 202 on a third parting surface 234 of a third block body 232 thereof. The third parting surface 234 is joined to the second lower parting surface 226 of the second block 220. Each molding recess 202 corresponds to the solution contact surface 14 of the solution contact portion 16 composing the gasket body 12 together with the slide contact portion 18.

The mold 200 described above is prepared and the uncut PTFE film 40, having a greater width than the aligned molding recesses 202, is arranged and set with respect to the molding recesses 202. Along with this, the molding elastomer blocks 204 are preliminarily fitted into the molding elastomer block fitting holes 228 of the second block 220, respectively.

It should be noted that "adhesiveness improving treatment" is required to be performed in advance for a surface of the uncut PTFE film 40 (the upper surface thereof in FIG. 5) that contacts each unmolded gasket body 12. This is because in general, the uncut PTFE film 40 is hardly adhesive and exerts a quite weak adhesive force when adhered to a vulcanized molded rubber, a thermoplastic elastomer, or so forth. This is also true of when the material of each unmolded gasket body 12 is the vulcanized molded rubber, the thermoplastic elastomer, or so forth as well as when the material of each unmolded gasket body 12 is "silicone rubber" described above.

Specifically, "adhesiveness improving treatment" can be exemplified by a method of disposing a silica fine particle layer on a joint surface between the uncut PTFE film 40 and each unmolded gasket body 12, chemical treatment with metallic sodium, plasma treatment performed in an argon atmosphere, or so forth. Furthermore, a mixture gas of oxygen and an easily available 1, 2-butadiene or 1, 3-butadiene gas may be introduced into a vacuum chamber in which the uncut PTFE film 40 is arranged and set, and subsequently, butadiene may be plasma-polymerized on the surface of the uncut PTFE film 40 using plasma.

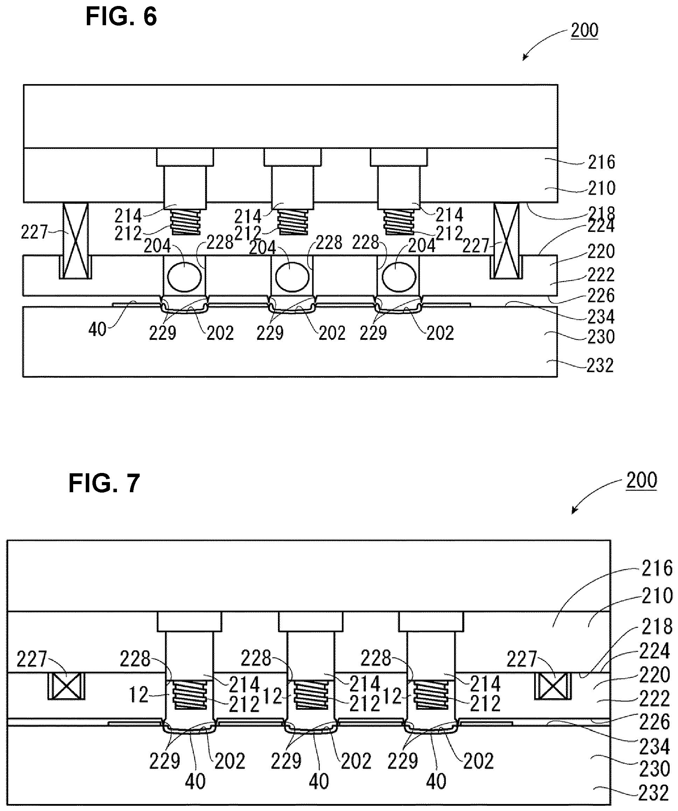

Thereafter, as shown in FIG. 6, the springs 227 are gradually pressed downward by the first parting surface 218 of the first block 210, whereby the second lower parting surface 226 of the second block 220 is gradually moved closer to the vicinity of the third parting surface 234 of the third block 230. Accordingly, the cutting portions 229, provided on the second lower parting surface 226, are pressed in contact with the uncut PTFE film 40 with a predetermined strength by the reaction forces of the springs 227. It should be noted that in this phase, the uncut PTFE film 40 has not been completely cut yet by the cutting portions 229 provided on the second lower parting surface 226. Instead of the springs 227, for instance, one or more hydraulic cylinders or so forth may be configured to act on the second block 220 such that the second lower parting surface 226 is gradually moved closer to the vicinity of the third parting surface 234, whereby the cutting portions 229 are pressed in contact with the uncut PTFE film 40 with a predetermined strength.

Subsequently, as shown in FIG. 7, the first parting surface 218 of the first block 210 is contacted to the second upper parting surface 224 of the second block 220, and each unmolded gasket body 12 is pressed toward each molding recess 202 located thereunder in FIG. 7 at a high pressure (of e.g., 10 to several hundred kPa) by each pair of the piston 214 and the male threaded screw 212 of the first block 210. Accordingly, during molding from each molding elastomer block 204 to each gasket body 12, a portion of each molding elastomer block 204, corresponding to the solution contact portion 16 of each gasket body 12, presses the uncut PTFE film 40 against each molding recess 202 at a high pressure. Then, under the pressure, the second lower parting surface 226 of the second block 220 is further moved closer to the third parting surface 234 of the third block 230, whereby the uncut PTFE film 40 is cut along the lateral edge of the solution contact portion 16 of each unmolded gasket body 12 by each cutting portion 229 provided on the second lower parting surface 226.

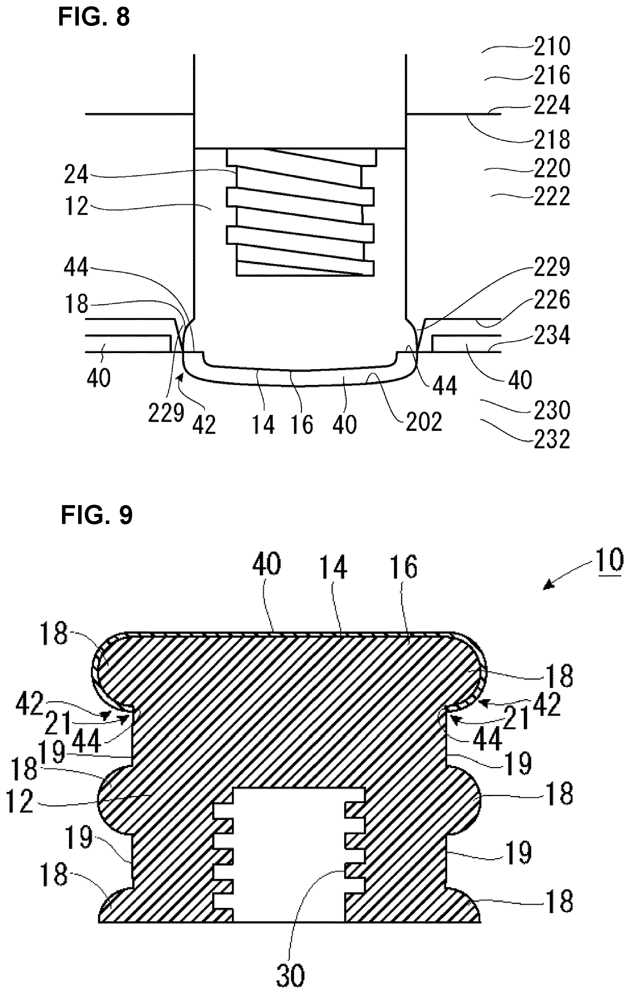

Thus, each unmolded gasket body 12 during molding and the uncut PTFE film 40 are pressed against each molding recess 202 at the high pressure, and simultaneously, the uncut PTFE film 40 is cut along the lateral edge of the solution contact portions 16 of each unmolded gasket body 12. Because of this, as shown in FIG. 8, the circumferential end portion 42 of the cut PTFE film 40 is stretched, while being sandwiched between the force (material fluid pressure) of each unmolded gasket body 12 (each molding elastomer block 204) attempting to expand along each molding recess 202 by the elasticity thereof and the reaction force from the lateral surface of each molding recess 202. Simultaneously, the circumferential end portion 42 of the cut PTFE film 40 is curved toward the slide contact portion 18 of each unmolded gasket body 12, while covering the circumferential edge of the solution contact surface 14 of each unmolded gasket body 12. Furthermore, the circumferential end surface 44 (cut surface) of the cut PTFE film 40 is buried in the slide contact portion 18 of each unmolded gasket body 12 so as not to be exposed to the outside and is made sealed by each unmolded gasket body 12.

Besides, according to the method of manufacturing the gasket 10 of the present preferred embodiment, joining of each unmolded gasket body 12 and the uncut PTFE film 40 and cutting of the uncut PTFE film 40 can be completed in a single processing step.

Furthermore, in comparison between pre and post manufacturing states of each gasket 10, an extension ratio Z of the circumferential end portion of the cut PTFE film 40 attached to the solution contact surface 14 of each gasket body 12 is less than or equal to 10%. In more detail, it is preferred to set the extension ratio Z to be less than or equal to 5%. Throughout the specification of the present application, the term "extension ratio Z" refers to a value calculated based on a ratio of an area X to an area Y, where the area X is defined as the area of the solution contact surface 14, to which the cut PTFE film 40 is attached, in each gasket body 12 (i.e., the area of the cut PTFE film 40 stretched in the manufacturing process of each gasket 10), whereas the area Y is defined as the area of a part of the uncut PTFE film 40 not attached yet, as the cut PTFE film 40, to each gasket body 12. The extension ratio Z can be expressed by the following formula: (1 - (X/Y)) × 100 = Z.

Finally, each gasket body 12 and the cut PTFE film 40 are taken out from each molding recess 202 in the mold 200, whereby manufacturing each gasket 10 is completed.

### (Features of Gasket 10)

(1) According to the gasket 10 of the present practical example, the circumferential end portion 42 of the cut PTFE film 40 is curved toward the slide contact portion 18 of the gasket body 12, and the circumferential end surface 44 of the cut PTFE film 40 is buried in the slide contact portion 18 of the gasket body 12 so as not to be exposed to the outside. Thus, the circumferential end surface 44 of the cut PTFE film 40 is not exposed to the outside, whereby it is possible to prevent occurrence of an undesirable situation that the medical solution 50 penetrates communicating holes existing inside and on the surface of the circumferential end surface 44 and this brings about leakage of the medical solution 50 from the gasket 10.
   Accordingly, it is possible to provide the gasket 10, by which undesirable leakage of the medical solution 50 is unlikely to occur when the gasket 10 is set in the syringe 100 and contacts the medical solution 50 over a long period of time.
(2) Besides, the gasket body 12 is made of silicone rubber provided with slidability. Hence, when the gasket body 12 is pressed in the mold 200 at a high pressure, silicone seeps from the silicone rubber, of which the gasket body 12 is made, and penetrates fine closed cells existing in the cut PTFE film 40. It is thereby possible to avoid occurrence of a situation that the medical solution 50 enters the closed cells and this finally brings about leakage of the medical solution 50. Because of this, probabilities of leakage of the medical solution 50 can be reduced as much as possible.
   Obviously, the silicone slightly seeps from the surface of the slide contact portion 18 of the gasket body 12 as well. Hence, the gasket body 12 can keep exerting excellent slidability against the inner surface 62 of the syringe barrel 60. Because of this, it is not required to apply a silicone oil to the inner surface 62 of the syringe barrel 60, unlike when a vulcanized molded rubber, a thermoplastic elastomer, or so forth is used as the material of the gasket body 12.
(3) As described above, the gasket body 12 during molding and the uncut PTFE film 40 are pressed against each molding recess 202 at a high pressure, and simultaneously, the uncut PTFE film 40 is cut along the lateral edge of the solution contact portion 16 of the molded gasket body 12. Accordingly, the circumferential end portion 42 of the cut PTFE film 40 is stretched, while being sandwiched between the force (material fluid pressure) of the gasket body 12 (each molding elastomer block 204) attempting to expand along each molding recess 202 by the elasticity thereof and the reaction force from the lateral surface of each molding recess 202. Simultaneously, the circumferential end portion 42 is curved toward the slide contact portion 18 of the gasket body 12, while covering the circumferential edge of the solution contact surface 14 of the gasket body 12. Furthermore, the circumferential end surface 44 (cut surface) of the cut PTFE film 40 is buried in the slide contact portion 18 of the gasket body 12 so as not to be exposed to the outside and is made sealed by the gasket body 12.

Here, a large number of closed cells, existing inside and on the surface of the cut PTFE film 40, are less likely to be connected to each other in alignment and become the communicating holes than in the prior arts, because the circumferential end portion 42 of the cut PTFE film 40 is set to have a low extension ratio (of less than or equal to 10%). Besides, the closed cells existing in the circumferential end portion 42 are collapsed between the gasket body 12 and each molding recess 202 at a high pressure in the manufacturing process of the gasket 10. Because of the above, the medical solution 50 becomes unlikely to enter the closed cells, whereby it is possible to reduce as much as possible the probabilities that the medical solution 50 penetrates the closed cells and leaks out.

### (Modification 1)

The shape and the manufacturing procedure of the gasket 10 may be configured as follows instead of those according to the preferred embodiment described above.

The gasket body 12 and the cut PTFE film 40, composing the gasket 10 according to a modification 1, are shaped as shown in FIG. 9. The gasket body 12 has an approximately columnar shape and is provided with plural protrusive cross-sectional slide contact portions 18 on the lateral surface thereof. Each protrusive cross-sectional slide contact portion 18 has an approximately semicircular cross section. It should be noted that each protrusive cross-sectional slide contact portion 18 is not limited to have the approximately semicircular cross section. Additionally or alternatively, the number of the protrusive cross-sectional slide contact portions 18 is not required to be plural; among the slide contact portions 18, only the one continuing immediately next to the solution contact portion 16 may be provided.

Among the slide contact portions 18, the closest one to the solution contact portion 16 is formed in continuation to the solution contact portion 16. Obviously, apexes of the slide contact portions 18 are each set to have a diameter slightly greater than a diameter (inner diameter) of the inner surface 62 of the syringe barrel 60 into which the gasket 10 is fitted. Besides, small diameter portions 19 are formed between the slide contact portions 18 adjacent to each other. Each small diameter portion 19 has a smaller diameter than each slide contact portion 18. When the gasket body 12 is seen in a cross-sectional view, each small diameter portion 19 has an approximately straight shape.

As similarly configured in the preferred embodiment described above, the cut PTFE film 40 is arranged and set to cover the solution contact surface 14 of the solution contact portion 16 of the gasket body 12. Besides, the circumferential end portion 42 of the cut PTFE film 40 extends across the apex of the slide contact portion 18 continuing immediately next to the solution contact portion 16 (toward the opposite side of the solution contact portion 16 from this slide contact portion 18) and is located on a boundary 21 between this slide contact portion 18 and the small diameter portion 19 continuing thereto. Accordingly, the circumferential end surface 44 of the cut PTFE film 40 is configured to face in surface contact with the lateral surface of the gasket body 12 (more specifically, a surface located in a position where the above-mentioned slide contact portion 18 ends and the small diameter portion 19 begins) on the boundary 21.

Based on the above, as similarly configured in the gasket 10 according to the preferred embodiment described above, the circumferential end surface 44 of the cut PTFE film 40 is not exposed to the outside. Hence, it is possible to prevent occurrence of the undesirable situation that when penetrating the cut PTFE film 40, the medical solution 50 leaks out from the circumferential end surface 44 through the communicating holes and this brings about leakage of the medical solution 50 from the gasket 10.

Next, the manufacturing procedure of the gasket 10 according to the modification 1 will be explained. The manufacturing procedure is basically the same as that in the preferred embodiment described above. Hence, the manufacturing procedure of the gasket 10 will be explained only when the contents thereof are different from those in the preferred embodiment described above; regarding the other contents, the explanation in the preferred embodiment will be incorporated herein by reference.

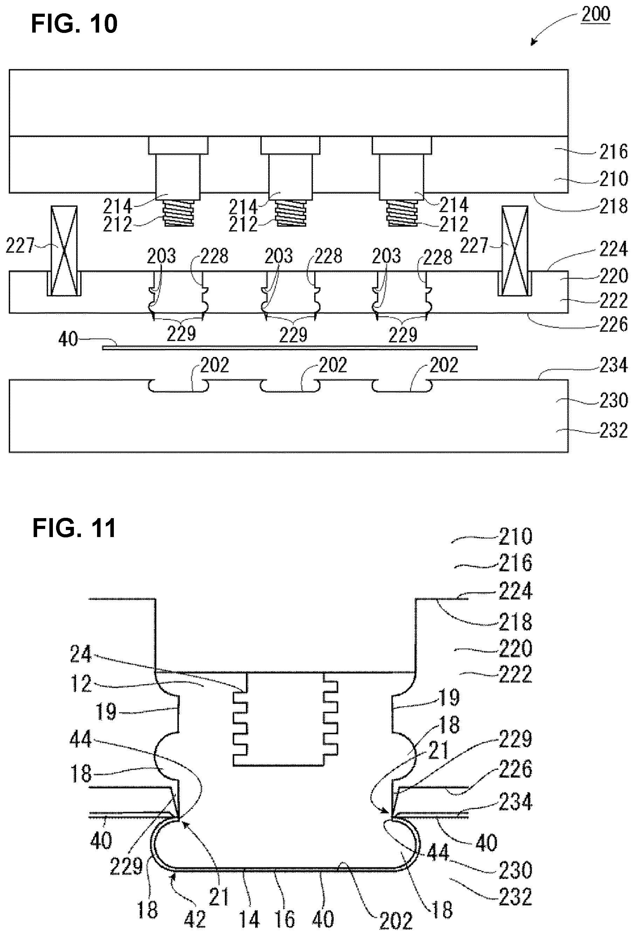

As shown in FIG. 10, the second block body 222 of the second block 220 includes the molding elastomer block fitting holes 228, each of which is provided with plural slide contact portion molding recesses 203 on the inner peripheral surface thereof. The slide contact portion molding recesses 203 correspond to the slide contact portions 18 except for the one continuing immediately next to the solution contact portion 16 of the gasket body 12.

Besides, the third block body 232 of the third block 230 is provided with the molding recesses 202 on the third parting surface 234 thereof joined to the second lower parting surface 226 of the second block 220. Each molding recess 202 corresponds to the solution contact portion 16 (the solution contact surface 14) and the slide contact portion 18 continuing immediately next to the solution contact portion 16 in the gasket body 12.

Then, each gasket body 12 during molding and the uncut PTFE film 40 are pressed against each molding recess 202 at a high pressure, and simultaneously, the uncut PTFE film 40 is cut on the boundary 21 between the slide contact portion 18 continuing immediately next to the solution contact portion 16 and the small diameter portion 19 continuing to this slide contact portion 18 in each gasket body 12. Accordingly, as shown in FIG. 11, the circumferential end portion 42 of the cut PTFE film 40 is stretched, while being sandwiched between the force of each gasket body 12 attempting to expand along each molding recess 202 by the elasticity thereof and the reaction force from the surface of each molding recess 202. Simultaneously, the circumferential end portion 42 is curved to the boundary 21, while covering the solution contact surface 14 and the slide contact portion 18 continuing immediately next to the solution contact portion 16 in each gasket body 12. Furthermore, the circumferential end surface 44 (cut surface) of the cut PTFE film 40 faces in surface contact with the lateral surface of each gasket body 12 (more specifically, a surface located in a position where the above-mentioned slide contact portion 18 ends and the above-mentioned small diameter portion 19 begins) on the boundary 21 so as not to be exposed to the outside.

### REFERENCE SIGNS LIST

10... Gasket, 12... Gasket body, 14...Solution contact surface, 16...Solution contact portion, 18...Slide contact portion, 19...Small diameter portion, 20...Small diameter portion, 21...Boundary, 22...Rear end surface, 24... Female threaded hole, 40...PTFE film, 42... Circumferential end portion, 44... Circumferential end surface, 50...Medical solution, 60...Syringe barrel, 62...Inner surface (of syringe barrel 60), 64...Barrel body, 66... Attached portion, 68...Flange portion, 70...Plunger rod, 72...Male threaded portion, 74...Finger pushing portion, 80...Top cap, 82...Cap body, 84...Cap flange portion, 86...Recess, 100...Syringe, 200...Mold, 202...Molding recess, 203...Slide contact portion molding recess, 204...Molding elastomer block, 210... First block, 212...Male threaded screw, 214... Piston, 216...First block body, 218...First parting surface, 220... Second block, 222...Second block body, 224...Second upper parting surface, 226...Second lower parting surface, 227...Spring, 228...Molding elastomer block fitting hole, 229... Cutting portion, 230...Third block, 232...Third block body, 234... Third parting surface

## Claims

1. A gasket (10) comprising:
a gasket body (12) including a solution contact portion (16) and a slide contact portion (18) continuing to the solution contact portion (16), the slide contact portion (18) having a protrusive cross section; and
a polytetrafluoroethylene (PTFE) film (40) attached to a solution contact surface (14) of the solution contact portion (16) of the gasket body (12),
wherein a circumferential end portion (42) of the PTFE film (40) is curved from the solution contact portion (16) of the gasket body (12) so as to extend across an apex of the slide contact portion (18) immediately next to the solution contact portion (16), while a circumferential end surface (44) of the PTFE film (40) faces in surface contact with a lateral surface of the gasket body (12) on a boundary (21) between the slide contact portion (18) and a small diameter portion (19) adjacent thereto.

2. The gasket (10) according to claim 1, wherein the circumferential end surface (44) of the PTFE film (40) is made sealed by the gasket body (12).

3. The gasket (10) according to any one of claims 1 to 2, wherein the circumferential end portion (42) of the PTFE film (40) attached to the solution contact surface (14) of the gasket body (12) has an extension ratio of less than or equal to 10%.

4. The gasket (10) according to any one of claims 1 to 3, wherein the gasket body (12) is made of silicone rubber provided with slidability.

5. A syringe (100) comprising:
the gasket (10) recited in any one of claims 1 to 4;
a medical solution (50);
a syringe barrel (60); and
a plunger rod (70).

6. A method of manufacturing a gasket (10), the method comprising:
preparing a mold (200) including a molding recess (202), the molding recess (202) provided in correspondence to a solution contact surface (14) of a solution contact portion (16) forming a gasket body (12) together with a slide contact portion (18);
arranging and setting a polytetrafluoroethylene (PTFE) film (40) with respect to the molding recess (202), the PTFE film (40) having a greater width than the molding recess (202);
during molding from a molding elastomer block (204) to the gasket body (12), pressing the PTFE film (40) to the molding recess (202) by a corresponding portion of the molding elastomer block (204) to the solution contact portion (16) of the gasket body (12); and
cutting the PTFE film (40) along a lateral edge of the solution contact portion (16) of the gasket body (12) under the pressure, whereby
a circumferential end portion (42) of the PTFE film (40) is curved to cover a circumferential edge of the solution contact surface (14), while a circumferential end surface (44) of the PTFE film (40) is buried in the slide contact portion (18) of the gasket body (12) so as not to be exposed to outside and is made sealed by the gasket body (12).

7. A method of manufacturing a gasket (10), the method comprising:
preparing a mold (200) including a molding recess (202), the molding recess (202) provided in correspondence to both a slide contact portion (18) and a solution contact surface (14) of a solution contact portion (16) forming a gasket body (12) together with the slide contact portion (18), the slide contact portion (18) continuing to the solution contact portion (16), the slide contact portion (18) having a protrusive cross section;
arranging and setting a polytetrafluoroethylene (PTFE) film (40) with respect to the molding recess (202), the PTFE film (40) having a greater width than the molding recess (202);
during molding from a molding elastomer block (204) to the gasket body (12), pressing the PTFE film (40) to the molding recess (202) by a corresponding portion of the molding elastomer block (204) to the slide contact portion (18) and the solution contact portion (16) of the gasket body (12); and
cutting the PTFE film (40) along a position located across an apex of the slide contact portion (18) in the gasket body (12) by closing the mold (200) in tight under the pressure, whereby
a circumferential end portion (42) of the PTFE film (40) is curved to cover a circumferential edge of the slide contact portion (18), while a circumferential end surface (44) of the PTFE film (40) faces in surface contact with a lateral surface of the gasket body (12) and is made sealed by the gasket body (12).

## Patentansprüche

1. Dichtung (10), aufweisend:
einen Dichtungskörper (12) mit einem Lösungskontaktabschnitt (16) und einem Gleitkontaktabschnitt (18), der sich an den Lösungskontaktabschnitt (16) anschließt, wobei der Gleitkontaktabschnitt (18) einen hervorstehenden Querschnitt aufweist; und
einen Polytetrafluorethylen (PTFE)-Film (40), der an einer Lösungskontaktfläche (14) des Lösungskontaktabschnitts (16) des Dichtungskörpers (12) angebracht ist,
wobei ein Umfangsendabschnitt (42) des PTFE-Films (40) von dem Lösungskontaktabschnitt (16) des Dichtungskörpers (12) so gekrümmt ist, dass er sich über einen Scheitelpunkt des Gleitkontaktabschnitts (18) unmittelbar neben dem Lösungskontaktabschnitt (16) erstreckt, während eine Umfangsendfläche (44) des PTFE-Films (40) in Oberflächenkontakt mit einer Seitenfläche des Dichtungskörpers (12) an einer Grenze (21) zwischen dem Gleitkontaktabschnitt (18) und einem daran angrenzenden Abschnitt (19) mit kleinem Durchmesser weist.

2. Dichtung (10) nach Anspruch 1, wobei die Umfangsendfläche (44) des PTFE-Films (40) durch den Dichtungskörper (12) abgedichtet wird.

3. Dichtung (10) nach einem der Ansprüche 1 bis 2, wobei der Umfangsendabschnitt (42) des PTFE-Films (40), der an der Lösungskontaktfläche (14) des Dichtungskörpers (12) angebracht ist, ein Dehnungsverhältnis von weniger als oder gleich 10 % aufweist.

4. Dichtung (10) nach einem der Ansprüche 1 bis 3, wobei der Dichtungskörper (12) aus Silikonkautschuk hergestellt ist, der Gleitfähigkeit aufweist.

5. Spritze (100), aufweisend:
die Dichtung (10) nach einem der Ansprüche 1 bis 4;
eine medizinische Lösung (50);
einen Spritzenzylinder (60); und
eine Kolbenstange (70).

6. Verfahren zum Herstellen einer Dichtung (10), wobei das Verfahren Folgendes aufweist:
Vorbereiten einer Form (200) mit einer Formaussparung (202), wobei die Formaussparung (202) in Übereinstimmung mit einer Lösungskontaktfläche (14) eines Lösungskontaktabschnitts (16) vorgesehen ist, der zusammen mit einem Gleitkontaktabschnitt (18) einen Dichtungskörper (12) bildet;
Anordnen und Festlegen eines Polytetrafluorethylen (PTFE)-Films (40) in Bezug auf die Formaussparung (202), wobei der PTFE-Film (40) eine größere Breite als die Formaussparung (202) aufweist;
während des Formens eines Formelastomerblocks (204) zu dem Dichtungskörper (12), Drücken des PTFE-Films (40) zu der Formaussparung (202) durch einen entsprechenden Abschnitt des Formelastomerblocks (204) zu dem Lösungskontaktabschnitt (16) des Dichtungskörpers (12); und
Schneiden des PTFE-Films (40) entlang einer Seitenkante des Lösungskontaktabschnitts (16) des Dichtungskörpers (12) unter dem Druck, wodurch
ein Umfangsendabschnitt (42) des PTFE-Films (40) so gekrümmt ist, dass er eine Umfangskante der Lösungskontaktfläche (14) bedeckt, während eine Umfangsendfläche (44) des PTFE-Films (40) in dem Gleitkontaktabschnitt (18) des Dichtungskörpers (12) so vergraben ist, dass sie nicht nach außen freiliegt und durch den Dichtungskörper (12) abgedichtet wird.

7. Verfahren zum Herstellen einer Dichtung (10), wobei das Verfahren Folgendes aufweist:
Vorbereiten einer Form (200) mit einer Formaussparung (202), wobei die Formaussparung (202) in Übereinstimmung mit sowohl einem Gleitkontaktabschnitt (18) als auch einer Lösungskontaktfläche (14) eines Lösungskontaktabschnitts (16) vorgesehen ist, der zusammen mit dem Gleitkontaktabschnitt (18) einen Dichtungskörper (12) bildet, wobei der Gleitkontaktabschnitt (18) sich an den Lösungskontaktabschnitt (16) anschließt, wobei der Gleitkontaktabschnitt (18) einen hervorstehenden Querschnitt aufweist;
Anordnen und Festlegen eines Polytetrafluorethylen (PTFE)-Films (40) in Bezug auf die Formaussparung (202), wobei der PTFE-Film (40) eine größere Breite als die Formaussparung (202) aufweist;
während des Formens eines Formelastomerblocks (204) zu dem Dichtungskörper (12), Drücken des PTFE-Films (40) zu der Formaussparung (202) durch einen entsprechenden Abschnitt des Formelastomerblocks (204) zu dem Gleitkontaktabschnitt (18) und dem Lösungskontaktabschnitt (16) des Dichtungskörpers (12); und
Schneiden des PTFE-Films (40) entlang einer Position, die sich über einen Scheitelpunkt des Gleitkontaktabschnitts (18) in dem Dichtungskörper (12) befindet, durch dichtes Schließen der Form (200) unter dem Druck, wodurch
ein Umfangsendabschnitt (42) des PTFE-Films (40) so gekrümmt ist, dass er eine Umfangskante des Gleitkontaktabschnitts (18) bedeckt, während eine Umfangsendfläche (44) des PTFE-Films (40) in Oberflächenkontakt mit einer Seitenfläche des Dichtungskörpers (12) weist und durch den Dichtungskörper (12) abgedichtet wird.

## Revendications

1. Joint d'étanchéité (10) comprenant :
un corps de joint d'étanchéité (12) comprenant une partie de contact de solution (16) et une partie de contact coulissante (18) continuant jusqu'à la partie de contact de solution (16), la partie de contact coulissante (18) ayant une section transversale en saillie ; et
un film de polytétrafluoroéthylène (PTFE) (40) fixé à une surface de contact de solution (14) de la partie de contact de solution (16) du corps de joint d'étanchéité (12),
dans lequel une partie d'extrémité circonférentielle (42) du film de PTFE (40) est incurvée à partir de la partie de contact de solution (16) du corps de joint d'étanchéité (12) de manière à s'étendre à travers un sommet de la partie de contact coulissante (18) immédiatement à côté de la partie de contact de solution (16), tandis qu'une surface d'extrémité circonférentielle (44) du film de PTFE (40) est orientée en contact de surface avec une surface latérale du corps de joint d'étanchéité (12) sur une limite (21) entre la partie de contact coulissante (18) et une partie de petit diamètre (19) adjacente à celle-ci.

2. Joint d'étanchéité (10) selon la revendication 1, dans lequel la surface d'extrémité circonférentielle (44) du film de PTFE (40) est rendue étanche par le corps de joint d'étanchéité (12).

3. Joint d'étanchéité (10) selon l'une quelconque des revendications 1 à 2, dans lequel la partie d'extrémité circonférentielle (42) du film de PTFE (40) fixée à la surface de contact de solution (14) du corps de joint d'étanchéité (12) a un rapport d'extension inférieur ou égal à 10 %.

4. Joint d'étanchéité (10) selon l'une quelconque des revendications 1 à 3, dans lequel le corps de joint d'étanchéité (12) est réalisé en caoutchouc de silicone doté d'une capacité de glissement.

5. Seringue (100) comprenant :
le joint d'étanchéité (10) selon l'une quelconque des revendications 1 à 4 ;
une solution médicale (50) ;
un cylindre de seringue (60) ; et
une tige de piston (70).

6. Procédé de fabrication d'un joint d'étanchéité (10), le procédé comprenant :
la préparation d'un moule (200) comprenant un évidement de moulage (202), l'évidement de moulage (202) prévu en correspondance avec une surface de contact de solution (14) d'une partie de contact de solution (16) formant un corps de joint d'étanchéité (12) conjointement avec une partie de contact coulissante (18) ;
l'agencement et la fixation d'un film de polytétrafluoroéthylène (PTFE) (40) par rapport à l'évidement de moulage (202), le film de PTFE (40) ayant une plus grande largeur que l'évidement de moulage (202);
pendant le moulage à partir d'un bloc élastomère de moulage (204) sur le corps de joint d'étanchéité (12), la pression du film de PTFE (40) sur l'évidement de moulage (202) par une partie correspondante du bloc élastomère de moulage (204) sur la partie de contact de solution (16) du corps de joint d'étanchéité (12) ; et
la coupe du film de PTFE (40) le long d'un bord latéral de la partie de contact de solution (16) du corps de joint d'étanchéité (12) sous la pression, moyennant quoi
une partie d'extrémité circonférentielle (42) du film de PTFE (40) est incurvée pour recouvrir un bord circonférentiel de la surface de contact de solution (14), tandis qu'une surface d'extrémité circonférentielle (44) du film de PTFE (40) est enfouie dans la partie de contact coulissante (18) du corps de joint d'étanchéité (12) de manière à ne pas être exposée à l'extérieur et est rendue étanche par le corps de joint d'étanchéité (12).

7. Procédé de fabrication d'un joint d'étanchéité (10), le procédé comprenant :
la préparation d'un moule (200) comprenant un évidement de moulage (202), l'évidement de moulage (202) prévu en correspondance à la fois avec une partie de contact coulissante (18) et une surface de contact de solution (14) d'une partie de contact de solution (16) formant un corps de joint d'étanchéité (12) conjointement avec la partie de contact coulissante (18), la partie de contact coulissante (18) continuant jusqu'à la partie de contact de solution (16), la partie de contact coulissante (18) ayant une section transversale en saillie ;
l'agencement et la fixation d'un film de polytétrafluoroéthylène (PTFE) (40) par rapport à l'évidement de moulage (202), le film de PTFE (40) ayant une plus grande largeur que l'évidement de moulage (202) ;
pendant le moulage à partir d'un bloc élastomère de moulage (204) sur le corps de joint d'étanchéité (12), la pression du film de PTFE (40) sur l'évidement de moulage (202) par une partie correspondante du bloc élastomère de moulage (204) sur la partie de contact coulissante (18) et la partie de contact de solution (16) du corps de joint d'étanchéité (12) ; et
la coupe du film de PTFE (40) le long d'une position située à travers un sommet de la partie de contact coulissante (18) dans le corps de joint d'étanchéité (12) en fermant le moule (200) de manière étanche sous la pression, moyennant quoi
une partie d'extrémité circonférentielle (42) du film de PTFE (40) est incurvée pour recouvrir un bord circonférentiel de la partie de contact coulissante (18), tandis qu'une surface d'extrémité circonférentielle (44) du film de PTFE (40) est orientée en contact de surface avec une surface latérale du corps de joint d'étanchéité (12) et est rendue étanche par le corps de joint d'étanchéité (12).
